# EUROPÄISCHE PATENTSCHRIFT

(11) **EP 2 214 596 B1**
(45) Veröffentlichungstag und Bekanntmachung des Hinweises auf die Patenterteilung: **30.01.2013**
(21) Anmeldenummer: 08857591.5
(22) Anmeldetag: 22.10.2008
(51) Int. Cl.: A61F 2/44

(54) **IMPLANTAT**
IMPLANT
IMPLANT

(30) Priorität: 05.12.2007 DE 102007058426
(43) Veröffentlichungstag der Anmeldung: 11.08.2010
(73) Patentinhaber: Tutogen Medical GmbH, 91077 Neunkirchen am Brand (DE)
(72) Erfinder: LANDIS, Klaus, 90562 Heroldsberg (DE)
(74) Vertreter: Manitz, Finsterwald & Partner GbR
(86) Internationale Anmeldenummer: PCT/EP2008/008935
(87) Internationale Veröffentlichungsnummer: WO 2009/071153

(56) Entgegenhaltungen:
- EP-A- 0 517 030
- WO-A-2005/074850
- US-A1- 2003 069 640
- US-A1- 2004 133 279
- US-B1- 6 902 578

## Beschreibung

Die vorliegende Erfindung betrifft ein zweiteiliges Implantat nach dem oberbegriff des Anspruchs 1.

Ein derartiges Implantat ist aus der US 2004/133279 A1 bekannt

Weiterhin sind einteilige Implantatformen oder auch Verbund-Implantate aus drei bis sechs verschiedenen Teilen bekannt. Derartige Implantate sind aufwendig herzustellen bzw. als einstückige Implantate aus natürlichem Knochenmaterial nur erschwert herstellbar.

Es ist die Aufgabe der Erfindung, ein Implantat der eingangs genannten Art zu schaffen, das einfach und kostengünstig herstellbar ist.

Die Lösung dieser Aufgabe erfolgt durch die Merkmale des Anspruchs 1 und insbesondere dadurch, dass jedes der beiden Teile mit einer Durchgangsöffnung versehen ist, wobei die Durchgangsöffnungen der beiden Teile miteinander fluchten. Weiterhin weisen die beiden Teile im Querschnitt gesehen entweder eine geschlossene oder eine einseitig offene Umfangskontur auf, wobei ein Teil eine Nut und das andere Teil eine korrespondierende Feder aufweist, die mit Presspassung ineinander gesteckt sind. Da ferner die Nut und die Feder der Umfangskontur der Teile folgen, ist eine außerordentlich feste Verbindung der beiden Teile zu einem Gesamtkörper gewährleistet, der sich bei Betrachtung mit bloßem Auge so gut wie nicht mehr von einem einstückigen Implantat unterscheidet.

Durch die erfindungsgemäße Merkmalskombination ist ein Implantat geschaffen, das aus vergleichsweise kleinvolumigem Knochenmaterial hergestellt werden kann, wobei das Zusammenfügen der beiden Implantatteile durch einfaches Ineinanderstecken erfolgt. Aufgrund des der Umfangskontur folgenden Verlaufs der Nut- und Federverbindung ist für eine feste Verbindung der Teile entlang der gesamten Umfangskontur gesorgt, ohne dass die beiden Teile mit zusätzlichen Bauteilen wie Stiften oder Pins oder dergleichen verbunden werden müssten.

Vorteilhafte Ausführungsformen der Erfindung sind in der Beschreibung, der Zeichnung sowie den Unteransprüchen beschrieben.

Nach einer ersten vorteilhaften Ausführungsform können die beiden Implantatteile gleichartig gestaltet sein, so dass sich nach dem Zusammenstecken der beiden Teile ein einheitlicher Gesamtkörper ergibt, der sich rein äußerlich fast nicht von einem einstückigen Implantatkörper unterscheiden lässt.

Nach einer weiteren vorteilhaften Ausführungsform kann in die Durchgangsöffnungen insbesondere formschlüssig der Einsatz aus spongiösem Knochen eingesetzt sein, der in den Durchgangsöffnungen mittels Presssitz bzw. Reibschluss gehalten ist, und der insbesondere die Durchgangsöffnungen vollständig ausfüllt. Ein solcher formschlüssiger Spongiosakern verbessert das Einwachsen des Implantats nach der Implantation.

Nach einer weiteren vorteilhaften Ausführungsform können die Außenflächen des Implantats im Bereich des Übergangs zwischen den beiden Teilen plan, d.h. ohne Stufen, erkennbare Rillen oder dergleichen verlaufen. Hierdurch besitzt das Implantat die gleichen günstigen Eigenschaften wie ein einstückiger Implantatkörper, wobei jedoch die beiden Teile des Implantats aus einem kleinvolumigeren Grundmaterial hergestellt werden können.

Es kann vorteilhaft sein, wenn die Durchgangsöffnungen einen Querschnitt besitzen, der nicht kreisförmig ist. Hierdurch ist für einen in die Durchgangsöffnung eingesetzten Einsatz eine Verdrehsicherung geschaffen.

Nach einer weiteren vorteilhaften Ausführungsform sind Nut und Feder derart gestaltet, dass die beiden Teile nur in einer einzigen relativen Orientierung zueinander ineinandersteckbar sind. Auf diese Weise wird ein versehentlich falsches Zusammenbauen der beiden Implantatteile verhindert.

Weiterhin kann es vorteilhaft sein, wenn die beiden Teile ausschließlich durch die Presspassung miteinander verbunden sind, da in diesem Fall auf weitere Hilfsmittel verzichtet werden kann. In bestimmten Anwendungsfällen kann jedoch das zusätzliche Verbinden der beiden Implantatteile mit Hilfe eines geeigneten Knochenklebers vorteilhaft sein.

Nachfolgend wird die vorliegende Erfindung rein beispielhaft anhand vorteilhafter Ausführungsformen und unter Bezugnahme auf die beigefügten Zeichnungen beschrieben. Es zeigen:
- Fig. 1: eine perspektivische Ansicht eines Implantatteils;
- Fig. 2: eine perspektivische Ansicht eines zu Fig. 1 komplementären Implantatteils;
- Fig. 3: eine Seitenansicht der ineinander gesteckten Implantatteile von Fig. 1 und Fig. 2;
- Fig. 4: eine Draufsicht auf das Implantat von Fig. 3 mit in die Durchgangsöffnungen eingesetztem Einsatz; und
- Fig. 5: eine Draufsicht auf eine nicht beanspruchte Ausführungsform eines Implantats mit Einsatz.

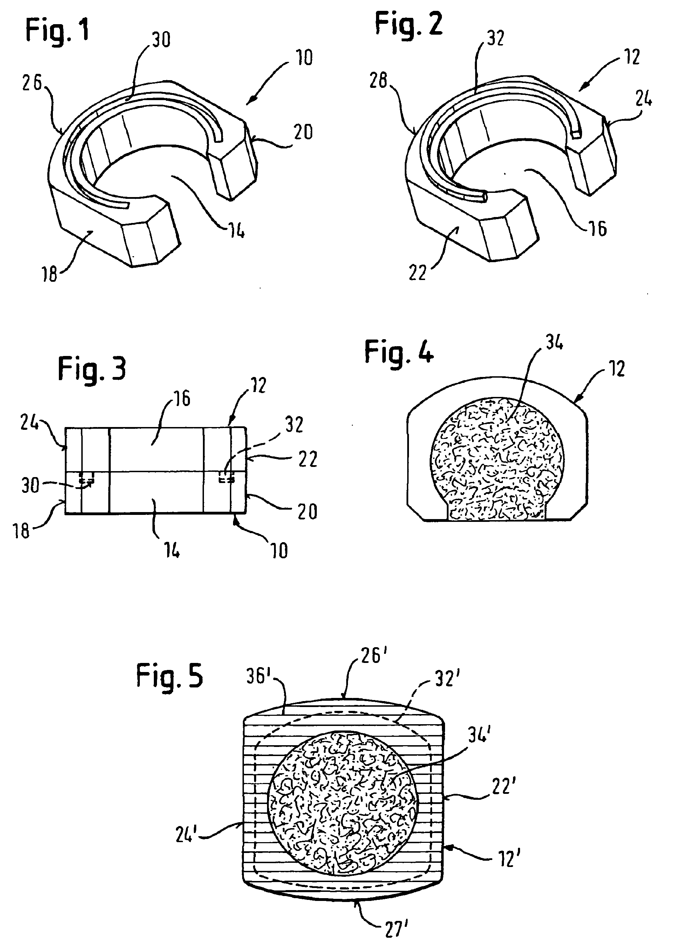

Die Fig. 1 und Fig. 2 zeigen zwei Teile 10 und 12 eines zweiteiligen Implantats, wobei beide Teile 10 und 12 aus bovinem oder humanem kortikalen Knochen hergestellt sind, der konserviert und sterilisiert ist. Jedes Implantatteil weist eine etwa hufeisenförmige Umfangskontur auf und ist mit einer Durchgangsöffnung 14 und 16 versehen, die von der Oberseite jedes Implantatteils zu dessen Unterseite reicht. An der in den Fig. 1 und Fig. 2 vorderen Seite der Implantatteile 10 und 12 sind diese ebenfalls aufgrund der Hufeisenform geöffnet, d.h. jedes Teil weist eine einseitig offene Umfangskontur auf. Die Seiten 18 und 20 des Teiles 10 sowie die Seiten 22 und 24 des Teiles 12 verlaufen zueinander parallel, wohingegen die jeweilige Rückseite 26 und 28 der beiden Implantatteile ballig gekrümmt ist.

An der Oberseite des Teils 10 ist eine Nut 30 ausgebildet, die der Umfangskontur des Teils 10 folgt, d.h. die sich annähernd C-förmig an der Oberseite des Teils 10 erstreckt. Das in Fig. 2 dargestellte Implantatteil 12 weist eine dazu korrespondierende bzw. komplementäre Feder 32 auf, die einstückig mit dem Teil 12 hergestellt ist und die ebenfalls der Umfangskontur des Teiles 12 folgt. Hierbei sind Nut 30 und Feder 32 so dimensioniert, dass die beiden Teile 10 und 12 mittels Presspassung ineinander gesteckt werden können.

Fig. 3 zeigt eine Vorderansicht der ineinander gesteckten Teile 10 und 12 von Fig. 1 und 2, wobei die Nut 30 sowie die Feder 32 strichpunktiert angedeutet sind. Da die einander zugewandten Oberflächen der Teile 10 und 12 außerordentlich plan gearbeitet sind und der Presssitz der der Umfangskontur der Teile folgenden Presspassung für eine sehr feste Verbindung der beiden Teile sorgt, lässt sich in zusammengestecktem Zustand der Teile 10 und 12 nicht einmal erkennen, dass es sich um ein zweiteiliges Implantat handelt.

Fig. 4 zeigt das Implantat von Fig. 3, wobei in die Durchgangsöffnungen 14 und 16 ein Einsatz 34 aus spongiösem Knochen eingesetzt ist, der in diesen mittels Presssitz bzw. Reibschluss gehalten ist. Der Einsatz 34 füllt dabei die Durchgangsöffnungen 14 und 16 vollständig aus und er schließt an der Vorderseite des Implantats bündig mit der Vorderseite der beiden Implantatteile 10 und 12 ab. Somit besitzt der Einsatz 34 eine annähernd zylindrische Form, wobei sich an diese zylindrische Grundform ein quaderförmiger Abschnitt einstückig anschließt, der den Raum zwischen den beiden Schenkelenden des Implantats ausfüllt.

Fig. 5 zeigt eine Draufsicht auf eine nicht beanspruchte Ausführungsform eines Implantats, wobei gleiche Bereiche mit gleichen, jedoch gestrichenen Bezugszeichen versehen sind.

Das in Fig. 5 dargestellte Implantat ist in gleicher Weise wie die vorstehend beschriebene Ausführungsform aus allogenem kortikalen Knochen hergestellt und besteht aus zwei Teilen, die über einen Presssitz miteinander verbunden sind. In der Draufsicht von Fig. 5 ist lediglich das eine Teil 12' zu erkennen, das mit einer ovalen Durchgangsöffnung versehen ist, in die ein Einsatz 34' aus spongiösem Knochen eingesetzt ist.

Die beiden Teile des Implantats von Fig. 5 weisen eine geschlossene Umfangskontur auf, die einen annähernd quadratischen Rahmen bildet, dessen gegenüberliegende Seiten 24' und 22' parallel verlaufen und dessen weitere gegenüberliegende Seiten 26' und 27' ballig ausgebildet sind. Im Unterschied zu der Ausführungsform der Figuren 1 bis 4 ist bei dieser Ausführungsform die in Fig. 5 erkennbare Oberseite (sowie die nicht erkennbare Unterseite) mit einer Parallelriffelung 36' versehen. Im Übrigen ist die in dem Teil 12' vorgesehene Nut 32' mit einer gestrichelten Linie angedeutet. Diese Nut 32' besitzt jedoch einen unsymmetrischen Verlauf im Bereich der balligen Abschnitte des Implantats, so dass die beiden Teile des Implantats nur ein einer einzigen Orientierung zueinander ineinander gesteckt werden können. Im Übrigen entspricht die Herstellung und die Montage dieser Ausführungsform der vorstehend beschriebenen. Die beschriebenen Implantate weisen eine Größe von etwa 11 x 11 bis 11 x 14 mm und im zusammengesetzten Zustand eine Höhe von etwa 6 bis 10 mm auf.

### Bezugszeichenliste

- 10, 12: Implantatteil
- 14, 16: Durchgangsöffnung
- 18 - 28: Außenfläche
- 22' - 27': Außenfläche
- 30: Nut
- 32, 32': Feder
- 34, 34': Einsatz
- 36': Riffelung

## Patentansprüche

1. Zweiteiliges Implantat aus bovinem oder humanem kortikalen Knochen, dessen beide Teile (10, 12,) über einen Presssitz miteinander verbunden sind, wobei
- jedes der beiden Teile (10, 12) mit einer Durchgangsöffnung (14, 16) versehen ist,
- die beiden Durchgangsöffnungen (14, 16) miteinander fluchten, und
- in die Durchgangsöffnungen (14, 16) ein Einsatz (34) aus spongiösem Knochen eingesetzt ist,
**dadurch gekennzeichnet, dass**
- die beiden Teile (10, 12) im Querschnitt eine einseitig offene, etwa hufeisenförmige Umfangskontur aufweisen,
- ein Teil (10) eine Nut (30) und der andere Teil (12) eine korrespondierende Feder (32) aufweist, die mit Presspassung ineinander gesteckt sind,
- wobei die Nut (30) und die Feder (32) der Umfangskontur der Teile folgen, und
- der Einsatz (34) in den Durchgangsöffnungen (14, 16) mittels Presssitz gehalten ist.

2. Implantat nach Anspruch 1,
**dadurch gekennzeichnet, dass**
die beiden Teile (10, 12) gleichartig gestaltet sind,

3. Implantat nach Anspruch 1 oder 2,
**dadurch gekennzeichnet, dass**
der Einsatz (34) die Durchgangsöffnungen (14, 16) vollständig ausfüllt.

4. Implantat nach zumindest einem der vorstehenden Ansprüche,
**dadurch gekennzeichnet, dass**
dessen Außenflächen (18-24) im Bereich des Übergangs zwischen den beiden Teilen (10, 12) plan verlaufen.

5. Implantat nach zumindest einem der vorstehenden Ansprüche,
**dadurch gekennzeichnet, dass**
die Durchgangsöffnungen (14, 16) einen Querschnitt besitzen, der nicht kreisförmig ist.

6. Implantat nach zumindest einem der vorstehenden Ansprüche,
**dadurch gekennzeichnet, dass**
Nut (30) und Feder (32) derart gestaltet sind, dass die beiden Teile (10, 12) nur in einer einzigen relativen Orientierung zueinander ineinander steckbar sind.

7. Implantat nach zumindest einem der vorstehenden Ansprüche,
**dadurch gekennzeichnet, dass**
dieses eine Grundfläche von mindestens 1 cm² aufweist.

8. Implantat nach zumindest einem der vorstehenden Ansprüche,
**dadurch gekennzeichnet, dass**
jedes Teil (10, 12) einstückig ausgebildet ist.

9. Implantat nach zumindest einem der vorstehenden Ansprüche,
**dadurch gekennzeichnet, dass**
die beiden Teile (10, 12) ausschließlich durch die Presspassung miteinander verbunden sind.

10. Implantat nach zumindest einem der vorstehenden Ansprüche,
**dadurch gekennzeichnet, dass**
der Einsatz (34) an der Vorderseite des Implantats bündig mit den beiden Teilen (10, 12) abschließt.

## Claims

1. A two-part implant of bovine or human cortical bone whose two parts (10, 12) are connected to one another via a press fit, wherein
- each of the two parts (10, 12) is provided with a passage opening (14, 16);
- the two passage openings (14, 16) are aligned with one another; and
- an inset (34) of spongious bone is inserted into the passage openings (14, 16),
**characterised in that**
- the two parts (10, 12) have a peripheral contour which is open at one side and of approximately sleeve shape in cross-section;
- one part (10) has a groove (30) and the other part (12) has a corresponding tongue (32) which are plugged into one another with a force fit,
- wherein the groove (30) and the tongue (32) follow the peripheral contour of the parts and
- the insert (34) is held in the passage openings (14, 16) by means of a press fit.

2. An implant in accordance with claim 1,
**characterised in that**
the two parts (10, 12) have a design of the same type.

3. An implant in accordance with claim 1 or claim 2,
**characterised in that**
the insert (34) completely fills the passage openings (14, 16).

4. An implant in accordance with at least one of the preceding claims,
**characterised in that**
its outer surfaces (18 - 24) extend in planar form in the region of the transition between the two parts (10, 12).

5. An implant in accordance with at least one of the preceding claims,
**characterised in that**
the passage openings (14, 16) have a cross-section which is not circular.

6. An implant in accordance with at least one of the preceding claims,
**characterised in that**
the groove (30) and tongue (32) are designed so that the two parts (10, 12) can only be plugged into one another in one single relative orientation towards one another.

7. An implant in accordance with at least one of the preceding claims,
**characterised in that**
it has a base surface of at least 1 cm².

8. An implant in accordance with at least one of the preceding claims,
**characterised in that**
each part (10, 12) is made in one piece.

9. An implant in accordance with at least one of the preceding claims,
**characterised in that**
the two parts (10, 12) are only connected to one another by the force fit.

10. An implant in accordance with at least one of the preceding claims,
**characterised in that**
the insert (34) terminates flush with the two parts (10, 12) at the front side of the implant.

## Revendications

1. Implant en deux parties à partir d'un os cortical bovin ou humain, dont les deux parties (10, 12) sont reliées l'une à l'autre par assemblage à la presse, dans lequel
- chacune des deux parties (10, 12) est dotée d'une ouverture traversante (14, 16),
- les deux ouvertures traversantes (14, 16) sont alignées l'une avec l'autre, et
- un insert (34) en os spongieux est mis en place dans les ouvertures traversantes (14, 16),
**caractérisé en ce que**
- les deux parties (10, 12) présentent en section transversale un contour périphérique ouvert d'un côté, approximativement en forme de fer à cheval,
- une partie (10) comporte une gorge (30) et l'autre partie (12) comporte une languette correspondante (32), qui sont enfichées l'une dans l'autre avec ajustement à la presse,
- dans lequel la gorge (30) et la languette (32) suivent le contour périphérique des parties, et
- l'insert (34) est maintenu dans les ouvertures traversantes (14, 16) au moyen d'un assemblage à la presse.

2. Implant selon la revendication 1,
**caractérisé en ce que** les deux parties (10, 12) sont conçues de manière égale.

3. Implant selon la revendication 1 ou 2,
**caractérisé en ce que** l'insert (34) remplit entièrement les ouvertures traversantes (14, 16).

4. Implant selon l'une au moins des revendications précédentes,
**caractérisé en ce que** ces surfaces extérieures (18-24) s'étendent de manière plane dans la région de la transition entre les deux parties (10, 12)

5. Implant selon l'une au moins des revendications précédentes,
**caractérisé en ce que** les ouvertures traversantes (14, 16) possèdent une section qui n'est pas circulaire.

6. Implant selon l'une au moins des revendications précédentes,
**caractérisé en ce que** la gorge (30) et la languette (32) sont conçues de telle façon que les deux parties (10, 12) sont enfichables l'une dans l'autre seulement dans une unique orientation relative l'une par rapport à l'autre.

7. Implant selon l'une au moins des revendications précédentes,
**caractérisé en ce que** celui-ci comporte une surface de base d'au moins 1 cm².

8. Implant selon l'une au moins des revendications précédentes,
**caractérisé en ce que** chaque partie (10, 12) est réalisée d'une seule pièce.

9. Implant selon l'une au moins des revendications précédentes,
**caractérisé en ce que** les deux parties (10, 12) sont reliées l'une à l'autre exclusivement par ajustement à la presse.

10. Implant selon l'une au moins des revendications précédentes,
**caractérisé en ce que** l'insert (34) se termine au niveau de la face antérieure de l'implant en affleurement avec les deux parties (10, 12).
